(19) Europäisches Patentamt
European Patent Office
Office européen des brevets

(11) **EP 2 693 206 B1**

## (12) EUROPÄISCHE PATENTSCHRIFT

(45) Veröffentlichungstag und Bekanntmachung des Hinweises auf die Patenterteilung:
**21.03.2018  Patentblatt 2018/12**

(51) Int Cl.:
*G01N 27/04* *(2006.01)*          *G01N 27/22* *(2006.01)*
*G01N 33/46* *(2006.01)*

(21) Anmeldenummer: **12178725.3**

(22) Anmeldetag: **31.07.2012**

(54) **Feuchtigkeitsmessgerät zur Wassergehaltsbestimmung von Biomasse**

Moisture meter for determining the water content of biomass

Appareil de mesure de l'humidité destiné à déterminer la teneur en eau de la biomasse

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(43) Veröffentlichungstag der Anmeldung:
**05.02.2014  Patentblatt 2014/06**

(73) Patentinhaber: **Messtechnik Schaller GmbH**
**8181 St. Ruprecht an der Raab (AT)**

(72) Erfinder: **Schaller, Maximilian**
**8200 Gleisdorf (AT)**

(74) Vertreter: **Schwarz & Partner Patentanwälte OG**
**Patentanwälte**
**Wipplingerstraße 30**
**1010 Wien (AT)**

(56) Entgegenhaltungen:
**EP-A1- 2 228 640     WO-A1-85/00427**
**CA-A- 1 080 305      US-A- 2 993 168**
**US-A- 3 025 465      US-A- 3 488 758**
**US-A- 4 021 733      US-A- 4 080 563**

## Beschreibung

Die Erfindung betrifft ein Messgerät zur Messung des Wassergehalts von Hackschnitzel mit einem Messbehälter zur Aufnahme der zu messenden Hackschnitzel und mit Messmitteln zur Einspeisung eines eine Messfrequenz aufweisenden Messsignals in die Hackschnitzel mittels Messelektroden und zur Messung des kapazitiven Verlustwiderstandes und mit einer Messgerätesteuerung zum Auswerten des gemessenen kapazitiven Verlustwiderstandes und zum Anzeigen des gemessenen Wassergehalts der Hackschnitzel und mit einer Komprimiereinheit zum Komprimieren der Hackschnitzel in dem Messbehälter vorgesehen ist.

Die Erfindung betrifft weiters ein Verfahren zur Messung des Wassergehalts von Hackschnitzel bei dem die folgenden Verfahrensschritte durchgeführt werden:

Einbringen der zu messenden Hackschnitzel in einen Messbehälter;
Einspeisen eines eine Messfrequenz aufweisenden Messsignals (MS) in die Hackschnitzel mittels Messelektroden;
Messen des kapazitiven Verlustwiderstandes;
Auswerten des gemessenen kapazitiven Verlustwiderstandes;
Anzeigen des gemessenen Wassergehalts der Hackschnitzel,

wobei die Hackschnitzel nach dem Einbringen in den Messbehälter und vor dem Messen des kapazitiven Verlustwiderstandes komprimiert werden.

Ein solches Messgerät zur Messung des Wassergehalts von Biomasse ist unter Produktbezeichnung " Humimeter BM2 " erhältlich. Dieses Messgerät ist beispielsweise zur Messung von Hackgut, Rinden, Pellets, Miscanthus, Maisspindel sowie Hobel- und Sägespänen vorgesehen. Zur Vorbereitung der Messung wird das Messgerät vorerst mit einem leeren Messbehälter kalibriert und dann wird die Biomasse von einem mitgelieferten Messeimer mit 13 Litern Inhalt langsam und gleichmäßig in den Messbehälter eingebracht. Hierbei muss auf eine gleichmäßige Schüttung und auf die Befüllung des Messbehälters mit einem bestimmten Gewicht der Biomasse geachtet werden. Je nach Art der Biomasse und je nach Gewicht der in den Messbehälter gefüllten Biomasse muss eine Kennlinie für die Auswertung durch die Messgerätesteuerung festgelegt werden.

Anschließend wird der Messvorgang gestartet, wobei das Messsignal zwischen plattenförmigen Elektroden die Biomasse durchläuft. Je nach Wassergehalt der Biomasse in dem Messbehälter bildet die Biomasse einen größeren oder einen kleineren kapazitiven Verlustwiderstand im Messkreis der Messmittel. Die Messgerätesteuerung des bekannten Messgeräts wertet den gemessenen kapazitiven Verlustwiderstand anhand der gewählten Kennlinie aus und zeigt den gemessenen Wassergehalt der Biomasse an einem Display des Messgeräts an.

Bei dem bekannten Messgerät hat sich als Nachteil ergeben, dass das Messergebnis relativ stark von der Schüttdichte der Biomasse in dem Messbehälter abhängt. Je geringer die Schüttdichte ist, desto mehr Luft befindet sich zwischen den Teilen der Biomasse und der gemessene kapazitive Verlustwiderstand wird durch diese Lufteinschlüsse stark beeinflusst. Benutzer des bekannten Messgeräts müssen daher die Biomasse bei jeder Messung möglichst gleichmäßig und immer gleich in den Messbehälter füllen, um die Messergebnisse vergleichen zu können und den Absolutwert des gemessenen Wassergehalts nicht zu verfälschen.

Dokument US 4,080,563 A offenbart ein Messgerät zur Messung des Wassergehalts von Futtermitteln, das eine manuelle Komprimiereinheit aufweist. Der Benutzer drückt das in einem auf Federn gelagerten Behälter enthaltene Futtermittel zusammen, worauf bei Vorliegen eines bestimmten immer gleichen Pressdrucks (Federkraft) ein elektrischer Kontakt geschlossen wird, durch den die kapazitive Messung des Wassergehalts aktiviert wird. Die kapazitive Messung an dem Futtermittel wird erst durch das Verdichten durch den Pressdruck ermöglicht.

Dokument US 2,993,168 A offenbart eine Komprimiereinheit für ein Messgerät zur Messung des Wassergehalts von Textilien, Chemikalien und Lebensmittel, die einen pneumatischen Zylinder aufweist, der das Material vor der Messung zusammen presst.

Dokument US 4,021,733 A offenbart ein Messgerät zur Messung des Wassergehalts von Korn und Heu, das eine manuelle Komprimiereinheit aufweist. Der Benutzer komprimiert die Biomasse mittels einer Schraube, worauf anschließend die kapazitive Messung des Wassergehalts durchgeführt wird.

Dokument US 2,025,465 A offenbart ein Messgerät zur Messung des Wassergehalts von Wolle, bei dem die Wolle, Cashmere oder ähnliche Fasern von einem Kolben in einem Zylinder komprimiert werden. Bei dem offenbarten Messverfahren wird der Kolben in Schritten in den Zylinder gefahren und je Komprimierschritt eine Kapazitätsmessung durchgeführt. Aus dieser Mehrzahl an Messungen kann anhand einer Tabelle der Wassergehalt der Wolle ermittelt werden.

Dokument WO 85/00427 A1 offenbart eine Wägezelle zur Messung des Gewichts von Korn, wobei das gemessene Gewicht wird zur Ermittlung des Wassergehalts unabhängig vom Gewicht verwendet wird.

CA1080305 offenbart ein auf Impedanzmessungen basierendes Messgerät zur rechnerischen Bestimmung des Wassergehalts, der Trockenmasse und Schüttdichte von Holzschnitzeln, deren Werte dafür verwendet werden das Mischungsverhältnis Wasser/Holzschnitzel zur Papierherstellung genau einzustellen. An einem Display werden die Temperatur und die Schüttdichte angezeigt.

Der Erfindung liegt die Aufgabe zugrunde ein Messgerät und ein Verfahren zur Messung des Wassergehalts von Hackschnitzeln zur Verfügung zu stellen, das einen Kennwert für den tatsächlichen Heizwert je Gewichtseinheit der gemessenen Hackschnitzel liefert. Erfindungsgemäß wird diese Aufgabestellung bei dem Messgerät dadurch gelöst, in dem Messgerät eine Wägezelle zur Messung des Gewichts der Hackschnitzel in dem Messbehälter vorgesehen ist und, dass die Messgerätesteuerung zum Anzeigen der Trockenmasse der gemessenen Hackschnitzel, also den Anteil der Hackschnitzel der kein Wasser enthält, ausgebildet ist.

Erfindungsgemäß wird diese Aufgabestellung bei dem Verfahren dadurch gelöst, das Gewicht der Hackschnitzel in dem Messbehälter gemessen wird und, dass die Trockenmasse der gemessenen Hackschnitzel, also den Anteil der Hackschnitzel der kein Wasser enthält, angezeigt wird.

Hierdurch ist der Vorteil erhalten, dass die Biomasse vor jeder Messung komprimiert wird, wodurch zumindest zwei wesentliche Vorteile erzielt werden. Einerseits ist die Schüttdichte somit bei jeder Messung vergleichbar, weshalb die Messergebnisse deutlich besser reproduzierbar sind. Andererseits nimmt durch das Komprimieren das Luftvolumen in der zu messenden Biomasse deutlich ab, weshalb der kapazitive Verlustwiderstand wesentlich stärker durch den unterschiedlichen Wassergehalt der Biomasse beeinflusst wird. Hierdurch kann die Genauigkeit der Messergebnisse deutlich gesteigert werden.

Als vorteilhaft hat sich weiters erwiesen einen bestimmten, gegebenenfalls von der jeweils zu messenden Biomasse abhängigen Komprimierdruck vorzugeben und diesen bei der Auswertung des gemessenen kapazitiven Verlustwiderstandes mit zu berücksichtigen. Hierdurch kann das Messergebnis noch weiter verbessert werden.

Weitere vorteilhafte Ausgestaltungen des erfindungsgemäßen Messgeräts und Verfahrens zur Messung des Wassergehalts von Biomasse werden im Folgenden anhand der Figuren näher erläutert.

Figur 1 zeigt ein Blockschaltbild der funktionalen Blöcke eines Messgeräts zur Messung des Wassergehalts von Biomasse.
Figur 2 zeigt ein Messgerät gemäß Figur 1 in einer Vorder- und einer Seitenansicht.
Figur 3 zeigt eine Schnittdarstellung des Messgeräts gemäß Figur 2 in einer Vorder- und einer Seitenansicht.

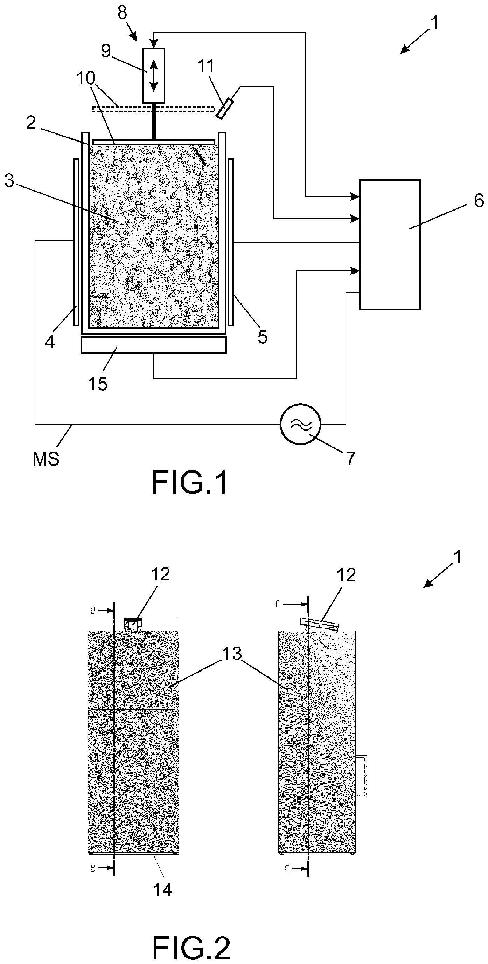

Figur 1 zeigt ein Blockschaltbild der funktionalen Blöcke eines Messgeräts 1 zur Messung des Wassergehalts von Biomasse. Das Messgerät 1 weist einen Messbehälter 2 zur Aufnahme der zu messenden Biomasse 3 auf. Als Biomasse 3 wird hierbei Hackgut, Rinden, Pellets, Miscanthus, Maisspindel sowie Hobel- und Sägespäne verstanden, wobei dem Fachmann auch andere kleinere Teile aufweisende durch natürliche oder teils auch künstliche Prozesse entstandene Materialien als Biomasse bekannt sind.

Das Messgerät 1 weist weiters Messmittel zur Einspeisung eines eine Messfrequenz aufweisenden Messsignals MS in die Biomasse 3 mittels Messelektroden 4 und 5 zur Messung des kapazitiven Verlustwiderstandes auf. Die Messelektroden 4 und 5 bilden hierbei einen Kondensator, in dem die Biomasse 3 als Dielektrikum enthalten ist. Die Messelektroden 4 und 5 sind in dem Blockschaltbild symbolisch als zwei an der Außenwand des Messbehälters 2 elektrisch isoliert vorgesehene Kondensatorplatten dargestellt. Dem Fachmann sind aber eine Vielzahl anderer Möglichkeiten bekannt Messelektroden zur Messung des kapazitiven Verlustwiderstands in der Biomasse 3 zu positioniert oder auszubilden.

Das Messgerät 1 weist weiters eine Messgerätesteuerung 6 zum Auswerten des gemessenen kapazitiven Verlustwiderstandes und zum Anzeigen des gemessenen Wassergehalts der Biomasse 3 an einem in Figur 1 nicht dargestellten Display auf. Die Messgerätesteuerung 6 ist zusätzlich zur Steuerung anderer Funktionen des Messgeräts 1 vorgesehen und durch einen Mikrocomputer realisiert. Eine Messsignalquelle 7 ist zum Erzeugen und Abgeben des eine Messfrequenz aufweisenden Messsignals MS an die Messelektrode 4 ausgebildet.

Das Messsignal MS durchläuft von der Messelektrode 4 die Biomasse 3 zur Messelektrode 5, worauf das durch den kapazitiven Verlustwiderstand beeinflusste Messsignal MS von der Messelektrode 5 an die Gerätesteuerung 6 zur Auswertung abgegeben wird. Die Messmittel zur Messung des kapazitiven Verlustwiderstandes sind somit durch die Messsignalquelle 7, die Messelektroden 4 und 5 und einen Teil der Gerätesteuerung 6 gebildet.

Das Messgerät 1 weist nunmehr eine Komprimiereinheit 8 zum Komprimieren der Biomasse 3 in dem Messbehälter 2 auf. Die Komprimiereinheit 8 ist durch einen Elektromotor 9 und eine Druckplatte 10 gebildet, die durch den Elektromotor 9 mit einem von der Gerätesteuerung 6 vorgegebenen Komprimierdruck auf die Biomasse 3 drückt. Anstatt des Elektromotors 9 könnte auch ein Hydraulik- oder Pneumatik-Zylinder vorgesehen sein.

Das Messgerät 1 weist nunmehr weiters eine in das Messgerät 1 integrierte Wägezelle 15 zur Messung des Gewichts der Biomasse 3 in dem Messbehälter 2 auf. Da das Gewicht der Biomasse 3 einen Einfluss auf die Auswertung des kapazitiven Verlustwiderstands durch die Gerätesteuerung 6 hat, muss der Gerätesteuerung 6 zur Ermittlung des Wassergehalts das Gewicht der Biomasse 3 in dem Messbehälter 2 bekannt sein. Diese Gewichtsmessung kann vor dem Einbringen der Biomasse 3 in den Messbehälter 2 erfolgen. Besonders vorteilhaft ist aber diese Gewichtsmessung mit der in das Messgerät 1 integrierten Wägezelle 15 durchzuführen, die im Zuge der Messung des kapazitiven Verlustwiderstands auch gleich das Gewicht misst und an die Gerätesteuerung 6 abgibt.

Das Messgerät 1 weist nunmehr weiters Temperaturmessmittel zur Messung der Temperatur der Biomasse in dem Messbehälter 2 auf, die durch ein IR-Thermometer 11 gebildet sind. Entweder vor dem Komprimieren oder, wenn die Druckplatte 10 nach dem Komprimieren von dem Elektromotor 9 wieder nach oben gefahren wurde, dann kann das IR-Thermometer 11 direkt an der Oberfläche der Biomasse 3 die Temperatur messen. Die Druckplatte 10 ist in dieser angehobenen Position strichliert in Figur 1 dargestellt. Vorteilhafterweise kann die Reproduzierbarkeit und die Messgenauigkeit des Messgeräts 1 durch die Berücksichtigung der Temperatur der Biomasse 3 bei der Ermittlung des Wassergehalts durch die Gerätesteuerung 6 weiter verbessert werden.

Zur Messung des Wassergehalts der Biomasse 3 mit dem Messgerät 1 wird das im Folgenden beschriebene Verfahren durchgeführt. Durch Tastendruck an Messgerät 1 wird dieses eingeschaltet und die Wägezelle mit dem Messbehälter 2 wird nach Abfrage am Display tariert. Bei einem ersten Verfahrensschritt wird der Messbehälter 2 aus dem Messgerät 1 entnommen, die zu messenden Biomasse 3 in den Messbehälter 2 eingebracht und anschließend der befüllte Messbehälter 2 wieder in das Messgerät 2 gestellt. Hierbei muss der Benutzer des Messgeräts 1 keine besondere Sorgfalt walten lassen. Anschließend wird am Messgerät 1 die Art der zu messenden Biomasse, also die Materialsorte, eingestellt und der Messvorgang mittels Taster gestartet.

Nun wird mittels des IR-Thermometers 11 die Temperatur der Biomasse in dem Messbehälter 2 gemessen. Bei einem zweiten Verfahrensschritt nach dem Einbringen der Biomasse 3 in den Messbehälter 2 und vor dem Messen des kapazitiven Verlustwiderstandes steuert die Gerätesteuerung 6 den Elektromotor 9 an und komprimiert die in dem Messbehälter 2 befindliche Biomasse 3 mit einem vorgegebenen Komprimierdruck bzw. bis ein bestimmtes Komprimiergewicht erreicht ist. Der vorzugebende Komprimierdruck kann beispielsweise etwa 500N betragen, ist aber von der Art der Biomasse und deren Teilchengröße und Festigkeit abhängig. Aus diesem Grund muss nach dem ersten Verfahrensschritt am Display des Messgeräts 1 vom Benutzer abgefragt werden, welche Art der Biomasse 3 gemessen werden soll. Je nach Eingabe durch den Benutzer wird der Komprimierdruck von der Gerätesteuerung 6 vorgegeben.

Bei einem dritten Verfahrensschritt wird das Messsignal MS über die Messelektroden 4 und 5 in die Biomasse 3 eingeleitet und der hierbei gemessene kapazitive Verlustwiderstand an die Gerätesteuerung 6 abgegeben. Bei der Auswertung des Messwertes zur Ermittlung des Wassergehalts der Biomasse 3 durch die Gerätesteuerung 6 benutzt die Gerätesteuerung 6 die jeweilige für die Art der Biomasse 3 typische Kennlinie. Die Kennlinie drückt hierbei den Zusammenhang zwischen gemessenem kapazitivem Verlustwiderstand und Wassergehalt der Biomasse 3 aus. Der von der Gerätesteuerung 6 anhand der ausgewählten Kennlinie ermittelte Wassergehalt kann anschließend am Display des Messgeräts 1 angezeigt werden.

Weiters wird von der Gerätesteuerung 6 die Schüttdichte und das Atro-Gewicht/$m^3$ Biomasse am Display 12 angezeigt. Hierbei wird die Schüttdichte vor dem Komprimieren der Biomasse 3 angezeigt, was insbesondere für jene Benutzer des Messgeräts 1 interessant ist, welche keine Brückenwaage zur Gewichtsermittlung der gelieferten Biomasse 3 zur Verfügung haben. In diesem Fall kann der Benutzer des Messgeräts 1 das Gewicht der gelieferten Biomasse 3 (z.B. des Hackgutes) über die Schüttdichte in Verbindung mit dem gelieferten Hackgutvolumen errechnen. Vorteilhaft kann es weiters sein, wenn die Gerätesteuerung 6 zusätzlich auch die Schüttdichte nach dem Komprimieren anzeigt. Diese Schüttdichte zeigt wie gut die Biomasse 3 in dem Messgerät 1 komprimiert werden konnte und diese Schüttdichte kann bei der Ermittlung des gemessenen Wassergehalts der Biomasse 3 berücksichtigt werden, worauf nachfolgend noch eingegangen ist.

Durch das Vorsehen der Komprimiereinheit 8 bei dem Messgerät 1 und durch das Vorsehen des zweiten Verfahrenschritts zum Komprimieren der Biomasse 3 vor der Messung ist der Vorteil erhalten, dass die Schüttdichte der Teilchen der Biomasse 3 erhöht wird. Einerseits ist die Schüttdichte somit bei jeder Messung vergleichbar, weshalb die Messergebnisse deutlich besser reproduzierbar sind. Andererseits nimmt durch das Komprimieren das Luftvolumen in der zu messenden Biomasse 3 deutlich ab, weshalb der kapazitive Verlustwiderstand wesentlich stärker durch den unterschiedlichen Wassergehalt der Biomasse 3 beeinflusst wird. Hierdurch kann die Genauigkeit der Messergebnisse deutlich gesteigert werden.

Als besonders vorteilhaft hat sich erwiesen, wenn die Gerätesteuerung 6 den bei der jeweiligen Messung vorgegebenen Kompressionsdruck bei der Ermittlung des Wassergehalts der Biomasse 3 mit berücksichtigt. Je größer der Kompressionsdruck beim Komprimieren war, desto größer ist die Schüttdichte und dieser Effekt wird bei der Ermittlung bzw. Berechnung des Wassergehalts durch die Gerätesteuerung 6 mit berücksichtigt. Dem Fachmann sind hierfür nötige Berechungen bekannt. Hierdurch kann die Genauigkeit der Messung weiter verbessert werden.

Als vorteilhaft hat sich weiters erwiesen mehrere Messungen des kapazitiven Verlustwiderstandes und der Temperatur der Biomasse 3 durchzuführen und die erhaltenen Messergebnisse oder den von der Gerätesteuerung 6 ermittelten Wassergehalt durch Mittelwertbildung besonders zuverlässig zu ermitteln und erst dann mit dem Display anzuzeigen.

Figur 2 zeigt eine Ausführungsform der funktionellen Blöcke des Messgeräts 1 gemäß Figur 1 in einer Vorder- und einer Seitenansicht. Das Gehäuse 12 des Displays in dem auch die Gerätesteuerung 6 untergebracht ist, ist oben am Gehäuse 13 des Messgeräts 1 zu sehen. An der Vorderseite des Gehäuses 13 des Messgeräts 1 ist eine Tür 14 zur Entnahme des Messbehälters 2 zu sehen.

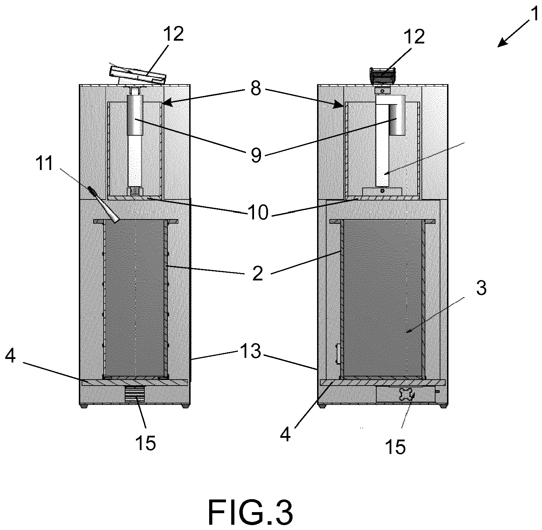

Figur 3 zeigt eine Schnittdarstellung des Messgeräts 1 gemäß Figur 2 in einer Vorder- und einer Seitenansicht. Die anhand von Figur 1 erläuterten funktionellen Blöcke sind mit gleichen Bezugszeichen auch in Figur 3 gekennzeichnet.

Die Messgerätesteuerung 6 des Messgeräts 1 ist weiters zum Anzeigen der Trockenmasse der gemessenen Biomasse 3 ausgebildet. Unter Trockenmasse oder Trockensubstanz (atro) versteht der Fachmann den Anteil der Biomasse der kein Wasser enthält. Dieser anerkannte Messwert ermöglicht eine unmittelbare Vergleichbarkeit des finanziellen Werts unterschiedlicher Biomassen der gleichen Art aber unterschiedlichen Wassergehalts. Ebenso kann es vorteilhaft sein die Schüttdichte unmittelbar am Messgerät 1 anzuzeigen.

Es kann erwähnt werden, dass es auch vorteilhaft sein kann die Biomasse 3 nicht nur vor der Messung sondern auch während der Messung des kapazitiven Verlustwiderstandes mit der Komprimiereinheit 8 zu komprimieren. Hierdurch kann der Anteil der Lufteinschlüsse in der Biomasse 3 während der Messung weiter reduziert werden.

Es kann erwähnt werden, dass in der Fachliteratur sowohl der Begriff des Wassergehalts als auch der Begriff des Feuchtegehalts von Biomasse verwendet wird. Hierbei entsprechen 100% Feuchtegehalt 50% Wassergehalt. Im Anschluss sind Formeln für die Berechnung des Wassergehalts und des Feuchtegehalts angegeben:

Bei der Definition des Wassergehalts wird die Feuchte entsprechend der Norm EN 14774 auf die Gesamtmasse bezogen berechnet:

$$\%WG = \frac{Mn - Mt}{Mn} \times 100$$

M$_n$: Masse der Probe vor dem Trocknen
M$_t$: Masse der getrockneten Probe
%WG: Errechneter Wassergehalt

Bei der Definition der Holzfeuchte (Feuchtegehalts) wird die Feuchte auf die Trockenmasse bezogen berechnet:

$$\%HF = \frac{Mn - Mt}{Mt} \times 100$$

%HF: Errechnete Holzfeuchte (Feuchtegehalt)

**Patentansprüche**

1. Messgerät (1) zur Messung des Wassergehalts von Hackschnitzel (3) mit einem Messbehälter (2) zur Aufnahme der zu messenden Hackschnitzel (3) und mit Messmitteln (4, 5, 6, 7) zur Einspeisung eines eine Messfrequenz aufweisenden Messsignals (MS) in die Hackschnitzel (3) mittels Messelektroden (4, 5) und zur Messung des kapazitiven Verlustwiderstandes und mit einer Messgerätesteuerung (6) zum Auswerten des gemessenen kapazitiven Verlustwiderstandes und zum Anzeigen des gemessenen Wassergehalts der Hackschnitzel (3) an einem Display der Messgerätesteuerung (6), und mit einer Komprimiereinheit (8) zum Komprimieren der Hackschnitzel (3) in dem Messbehälter (2) vorgesehen ist, wobei in dem Messgerät (1) eine Wägezelle (15) zur Messung des Gewichts der Hackschnitzel (3) in dem Messbehälter (2) vorgesehen ist und das Display der Messgerätesteuerung (6) die Trockenmasse der gemessenen Hackschnitzel (3), also den Anteil der Hackschnitzel (3) der kein Wasser enthält, anzeigt.

2. Messgerät (1) gemäß Anspruch 1, **dadurch gekennzeichnet, dass** die Komprimiereinheit (8) zum Komprimieren der Hackschnitzel (3) mit einem vorgegebenen Komprimierdruck ausgebildet ist.

3. Messgerät (1) gemäß Anspruch 2, **dadurch gekennzeichnet, dass** die Messgerätesteuerung (6) bei der Ermittlung des gemessenen Wassergehalts der Hackschnitzel (3) zum Berücksichtigen des Komprimierdrucks der Komprimiereinheit (8) ausgebildet ist.

4. Messgerät (1) gemäß einem der vorstehenden Ansprüche, **dadurch gekennzeichnet, dass** die Messgerätesteuerung (6) zum Auswerten mehrerer Messungen und zur Anzeige eines aus den Messungen gemittelten gemessenen Wassergehalts der Hackschnitzel (3) an dem Display ausgebildet ist.

5. Messgerät (1) gemäß einem der vorstehenden Ansprüche, **dadurch gekennzeichnet, dass** die Messgerätesteuerung (6) die Schüttdichte der Hackschnitzel (3) an dem Display vor dem Komprimieren und auch nach dem Komprimieren anzeigt.

6. Messgerät (1) gemäß Anspruch 5, **dadurch gekennzeichnet, dass** die Messgerätesteuerung (6) bei der Ermittlung des gemessenen Wassergehalts der Hackschnitzel (3) zum Berücksichtigen der durch die Komprimierung erreichten Schüttdichte der Hackschnitzel ausgebildet ist.

7. Messgerät (1) gemäß einem der vorstehenden Ansprüche, **dadurch gekennzeichnet, dass** Temperaturmessmittel zur Messung der Temperatur der Hackschnitzel (3) in dem Messbehälter (2) vorgesehen sind.

8. Messgerät (1) gemäß Anspruch 1, **dadurch gekennzeichnet, dass** die Messgerätesteuerung (6) bei der Ermittlung des gemessenen Wassergehalts der Hackschnitzel (3) zum Berücksichtigen der gemessenen Temperatur der Hackschnitzel (3) ausgebildet ist.

9. Verfahren zur Messung des Wassergehalts von Hackschnitzel (3) bei dem die folgenden Verfahrensschritte durchgeführt werden:

Einbringen der zu messenden Hackschnitzel (3) in einen Messbehälter (2);
Einspeisen eines eine Messfrequenz aufweisenden Messsignals (MS) in die Hackschnitzel (3) mittels Messelektroden (4, 5);
Messen des kapazitiven Verlustwiderstandes;
Auswerten des gemessenen kapazitiven Verlustwiderstandes;
Anzeigen des gemessenen Wassergehalts der Hackschnitzel (3) an einem Display der Messgerätesteuerung (6), wobei die Hackschnitzel (3) nach dem Einbringen in den Messbehälter (2) und vor dem Messen des kapazitiven Verlustwiderstandes komprimiert werden, **dadurch gekennzeichnet, dass**
das Gewicht der Hackschnitzel (3) in dem Messbehälter (2) gemessen wird und, dass die Trockenmasse der gemessenen Hackschnitzel (3), also den Anteil der Hackschnitzel (3) der kein Wasser enthält, an einem Display der Messgerätesteuerung (6) angezeigt wird.

10. Verfahren gemäß Anspruch 9, **dadurch gekennzeichnet, dass** zur Ermittlung des gemessenen Wassergehalts der Hackschnitzel (3) ein vorgegebener Komprimierdruck und die Temperatur der Hackschnitzel (3) berücksichtigt wird.

**Claims**

1. A meter (1) for measuring the water content of wood chips (3) using a metering box (2) for accommodating the wood chips (3) to be measured and having metering means (4, 5, 6, 7) for feeding a metering signal (MS) having a metering frequency into the wood chips (3) by means of metering electrodes (4, 5) and for measuring the capacitive loss resistance and having a meter control (6) for evaluating the capacitive loss resistance measured and for displaying the water content of the wood chips (3) measured on a display of the meter control (6) and having a compacting unit (8) for compacting the wood chips (3) in the metering box (2), wherein there is provided in the meter (1) a weighing cell (15) for measuring the weight of the wood chips (3) in the metering box (2) and wherein the display of the meter control (6) displays the dry mass of the wood chips (3) measured, this is the portion of the wood chips (3) not containing any water.

2. A meter (1) according to claim 1, **characterized in that** the compacting unit (8) is configured to compact the wood chips (3) at a predetermined compacting pressure.

3. A meter (1) according to claim 2, **characterized in that** the meter control (6) is configured to take into account the compacting pressure of the compacting unit (8) when determining the measured water content of the wood chips (3).

4. A meter (1) according to any of the preceding claims, **characterized in that** the meter control (6) is configured to evaluate a multiplicity of measurements and to display a measured water content of the wood chips (3) that is averaged from the measurements.

5. A meter (1) according to any of the preceding claims, **characterized in that** the meter control (6) displays the bulk density of the wood chips (3) on the display before compacting and also after compacting.

6. A meter (1) according to claim 5, **characterized in that** the meter control (6) is configured to take into account the bulk density of the wood chips (3) obtained by compacting when determining the measured water content of the wood chips (3).

7. A meter (1) according to any of the preceding claims, **characterized in that** there are provided temperature metering means for measuring the temperature of the wood chips (3) in the metering box (2).

8. A meter (1) according to claim 1, **characterized in that** the meter control (6) is configured to take into account the measured temperature of the wood chips (3) when determining the measured water content of the wood chips (3).

9. A method for measuring the water content of wood chips (3), wherein the following method steps are being carried out:

introducing the wood chips (3) to be measured into a metering box (2);
feeding a metering signal (MS) having a metering frequency into the wood chips (3) by means of metering electrodes (4, 5);
measuring the capacitive loss resistance;
evaluating the capacitive loss resistance measured;

displaying the measured water content of the wood chips (3) on a display of the meter control (6),

wherein the wood chips (3) are compacted after introduction into the metering box (2) and before the measurement of the capacitive loss resistance, **characterized in that**

the weight of the wood chips (3) in the metering box (2) is measured and that the dry mass of the measured wood chips (3), this is the portion of the wood chips (3) not containing any water, is displayed on a display of the meter control (6).

10. A method according to claim 9, **characterized in that** a predetermined compacting pressure and the temperature of the wood chips (3) are taken into account for determining the measured water content of the wood chips (3).


## Revendications

1. Appareil de mesure (1) pour mesurer la teneur en eau de copeaux de bois (3), comportant un récipient de mesure (2) pour recevoir les copeaux de bois à mesurer (3) et comportant des moyens de mesure (4, 5, 6, 7) pour injecter dans les copeaux de bois (3) un signal de mesure (MS) ayant une fréquence de mesure à l'aide d'électrodes de mesure (4, 5) et pour mesurer la résistance de perte capacitive, et comportant

   une commande d'appareil de mesure (6) pour évaluer la résistance de perte capacitive mesurée et pour afficher la teneur en eau mesurée des copeaux de bois (3) sur un écran d'affichage de la commande d'appareil de mesure (6) et comportant une unité de compactage (8) pour compacter les copeaux de bois (3) dans le récipient de mesure (2),

   dans lequel

   une cellule de pesage (15) destinée à mesurer le poids des copeaux de bois (3) dans le récipient de mesure (2) est prévue dans l'appareil de mesure (1) et l'écran d'affichage de la commande d'appareil de mesure (6) affiche la masse sèche des copeaux de bois mesurés (3), donc la part de copeaux de bois (3) qui ne contient pas d'eau.

2. Appareil de mesure (1) selon la revendication 1, **caractérisé en ce que** l'unité de compactage (8) pour compacter les copeaux de bois (3) est réalisée avec une pression de compactage prédéterminée.

3. Appareil de mesure (1) selon la revendication 2, **caractérisé en ce que** la commande d'appareil de mesure (6) est réalisée pour prendre en compte la pression de compactage de l'unité de compactage (8) lors de la détermination de la teneur en eau mesurée des copeaux de bois (3).

4. Appareil de mesure (1) selon l'une des revendications précédentes, **caractérisé en ce que** la commande d'appareil de mesure (6) est réalisée pour évaluer plusieurs mesures et pour afficher sur l'écran d'affichage une teneur en eau mesurée des copeaux de bois moyennée à partir des mesures.

5. Appareil de mesure (1) selon l'une des revendications précédentes, **caractérisé en ce que** la commande d'appareil de mesure (6) affiche sur l'écran d'affichage la densité en vrac des copeaux de bois (3) avant le compactage et également après le compactage.

6. Appareil de mesure (1) selon la revendication 5, **caractérisé en ce que** la commande d'appareil de mesure (6) est réalisée pour prendre en compte la densité en vrac des copeaux de bois atteinte par le compactage, lors de la détermination de la teneur en eau mesurée des copeaux de bois (3).

7. Appareil de mesure (1) selon l'une des revendications précédentes, **caractérisé en ce qu'**il est prévu des moyens de mesure de température pour mesurer la température des copeaux de bois (3) dans le récipient de mesure (2).

8. Appareil de mesure (1) selon la revendication 1, **caractérisé en ce que** la commande d'appareil de mesure (6) est réalisée pour prendre en compte la température mesurée des copeaux de bois (3) lors de la détermination de la teneur en eau mesurée des copeaux de bois (3).

9. Procédé pour mesurer la teneur en eau de copeaux de bois (3), dans lequel on met en oeuvre les étapes suivantes consistant à :

   introduire les copeaux de bois à mesurer (3) dans un récipient de mesure (2) ;
   injecter un signal de mesure (MS) présentant une fréquence de mesure dans les copeaux de bois (3) à l'aide d'électrodes de mesure (4, 5) ;
   mesurer la résistance de perte capacitive ;
   évaluer la résistance de perte capacitive mesurée ;
   afficher la teneur en eau mesurée des copeaux de bois (3) sur un écran d'affichage de la commande d'appareil de mesure (6),
   dans lequel on compacte les copeaux de bois (3) après les avoir introduits dans le récipient de mesure (2) et avant de mesurer la résistance de perte capacitive,
   **caractérisé en ce que**
   on mesure le poids des copeaux de bois (3) dans le récipient de mesure (2), et **en ce que** l'on affiche sur un écran d'affichage de la commande d'appareil de mesure (6) la masse sèche des copeaux de bois mesurés (3), donc la part des copeaux de bois (3) qui ne contient pas d'eau.

**10.** Procédé selon la revendication 9, **caractérisé en ce qu'**on prend en compte une pression de compactage donnée et la température des copeaux de bois (3) pour la détermination de la teneur en eau mesurée des copeaux de bois (3).

FIG.1

FIG.2

FIG.3

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 4080563 A
- US 2993168 A
- US 4021733 A
- US 2025465 A
- WO 8500427 A1
- CA 1080305